# EUROPEAN PATENT APPLICATION

(11) **EP 3 915 614 A1**
(43) Date of publication of application: **01.12.2021**
(21) Application number: 19911641.9
(22) Date of filing: 27.12.2019
(51) Int. Cl.: A61M 5/303, A61M 5/31, C08L 23/00, C08L 55/02, C08L 67/00, C08L 69/00, C08L 77/00

(54) **CRACK RESISTANT MEMBER**

(30) Priority: 25.01.2019 JP 2019011509
(71) Applicant: Daicel Corporation, Osaka 530-0011 (JP)
(72) Inventor: MORINAGA, Yasunori, Tokyo 108-8230 (JP)
(74) Representative: Grünecker Patent- und Rechtsanwälte PartG mbB
(86) International application number: PCT/JP2019/051524
(87) International publication number: WO 2020/153104

(57) **Abstract**

A crack resistant member including a member body, a first region (33) that is a hollow space provided in the member body to open to a surface of the member body and is configured to dispose a driving part that outputs a pressing force in an extension direction of the space, and a coupling portion that is configured to mechanically couple to the driving part and is formed on an inner wall surface of the first region (33), in which at least the coupling portion is formed from a resin material having a flexural modulus of 2600 MPa or greater as measured in accordance with ISO 178.

## Description

### Technical Field

The present disclosure relates to a crack resistant member and a needleless injector using the crack resistant member.

### Background Art

In resin products that receive an impact load, a crack may be generated at or near a portion where the impact load is applied, and the generated crack may grow, leading to occurrence of damage or breakage. Thus, safety issues such as scattering of fragments of a member and a problem of appearance failure have occurred, and attempts have been made to improve crack resistance of the resin products against impact load.

For example, Patent Document 1 discloses an impact resistant polyamide composition formed from a polyamide and an ionomer. However, when a large impact load is instantaneously applied to the thermoplastic composition, cracks are generated, and breakage occurs. Thus, there is a demand for development of a resin product having excellent crack resistance against an instantaneous and large load impact.

### Citation List

### Patent Document

Patent Document 1: JP 51-151797 A

### Summary of Invention

### Technical Problem

In light of the problems described above, it is an object of the present disclosure to provide a crack resistant member that can suppress crack generation and crack growth even when an impact load is applied, especially even when a large impact load is applied instantaneously.

### Solution to Problem

As a result of diligent research, the present inventors have found that crack generation and crack growth can be suppressed by forming a site where at least an impact load is applied by a resin material having a predetermined flexural modulus, so that the above problems can be solved. That is, the present disclosure is as described below.

<1>
   A crack resistant member including
   a member body,
   a first region that is a hollow space provided in the member body to open to a surface of the member body and is configured for disposing a driving part that outputs a pressing force in an extension direction of the space, and
   a coupling portion that is configured for mechanically coupling to the driving part and is formed on an inner wall surface of the first region,
   in which at least the coupling portion is formed from a resin material having a flexural modulus of 2600 MPa or greater as measured in accordance with ISO 178.
<2>
   The crack resistant member described in <1>, in which the driving part includes a drive source that contains predetermined energy for generating the pressing force, and is configured to generate the pressing force by releasing the predetermined energy to outside.
<3>
   The crack resistant member described in <2>, in which the drive source is an ignition charge, and
   the driving part is configured to generate the pressing force by releasing combustion energy which is caused by combustion of the ignition charge and is the predetermined energy.
<4>
   The crack resistant member described in <2> or <3>, in which the driving part is configured to complete the release of the predetermined energy within 10 msec from start of actuation of the driving part.
<5>
   The crack resistant member described in any one of <2> to <4>, in which the coupling portion is a screw portion formed on the inner wall surface of the first region along an extension direction of the first region.
<6>
   The crack resistant member described in any one of <1> to <5>, in which the resin material contains one or more selected from the group consisting of a mixture of a polycarbonate resin and an acrylonitrile-butadiene-styrene copolymer (ABS resin), a polyolefin-based resin, a crystalline polyamide resin, and a crystalline polyester resin.
<7>
   The crack resistant member described in <6>, in which in the mixture of the polycarbonate resin and the ABS resin, a proportion of the ABS resin relative to a total amount of the polycarbonate resin and the ABS resin is 10 mass% or greater and 30 mass% or less.
<8>
   A needleless injector configured to eject an intended injection substance to a target region without using an injection needle, the needleless injector including
   an attachment that is the crack resistant member described in any one of <1> to <7> and couples an actuator, which is the driving part, into the attachment with the coupling portion,
   the actuator,
   a housing part that has an accommodating space that accommodates the intended injection substance and defines a flow path from the accommodating space in such a manner that the intended injection substance is ejected to the target region,
   a metal piston which is arranged to propel inside the needleless injector in a predetermined direction when the pressing force is applied by actuation of the driving part, and
   a plunger that defines the accommodating space and propels inside the housing part by the pressing force to pressurize the intended injection substance.

### Advantageous Effects of Invention

Provided is a crack resistant member that suppresses crack generation and crack growth even when an impact load is applied, especially even when a large impact load is applied instantaneously.

### Brief Description of Drawings

FIG. 1 is a diagram illustrating a schematic configuration of a needleless injector.
FIG. 2 is a first cross-sectional view of a needleless injector.
FIG. 3 is a second cross-sectional view of the needleless injector.
FIG. 4 is a diagram illustrating a configuration of a housing of the needleless injector.
FIG. 5 is a diagram illustrating a schematic configuration of an injector assembly incorporated in the needleless injector.
FIG. 6 is a diagram illustrating a schematic configuration of an actuator incorporated in the needleless injector.
FIG. 7 is a diagram illustrating a schematic configuration of a piston incorporated in the needleless injector.
FIG. 8 is a diagram illustrating a schematic configuration of an attachment incorporated in the needleless injector.
FIG. 9 is a diagram illustrating a schematic configuration of a plunger rod and plunger incorporated in the needleless injector.
FIG. 10 is a diagram illustrating a schematic configuration of a container incorporated in the needleless injector.

### Description of Embodiments

Hereinafter, although the present disclosure will be described in detail, each of the configurations, combinations thereof, and the like in each embodiment are an example, and various additions, omissions, substitutions, and other changes may be made as appropriate without departing from the spirit of the present invention. The present disclosure is not limited by the embodiments and is limited only by the claims.

Also, each aspect disclosed in the present specification can be combined with any other feature disclosed herein.

### <Crack resistant member>

In a crack resistant member according to an embodiment of the present disclosure, a configuration is employed in which at least a site where an impact load is applied is formed from a resin material having a predetermined flexural modulus, so that crack generation and crack growth are suppressed. Such a configuration allows crack generation and crack growth to be suppressed even when an impact load is applied to a coupling portion, especially even when a relatively large impact load is applied within a short period of time.

Specifically, the crack resistant member according to the present embodiment is a crack resistant member including a member body, a first region that is a hollow space provided in the member body to open to a surface of the member body and is configured for disposing a driving part that outputs a pressing force in an extension direction of the space, and a coupling portion that is configured for mechanically coupling to the driving part and is formed on an inner wall surface of the first region. In this crack resistant member, at least the coupling portion is formed from a resin material having a flexural modulus of 2600 MPa or greater as measured in accordance with ISO 178. According to such a configuration, when the driving part outputs the pressing force in an extension direction of the first region, an impact load in a direction opposite the extension direction is applied to the coupling portion.

In the crack resistant member according to the present embodiment, the first region extends from an opening portion of the surface of the member body to the center. The crack resistant member is used in a state in which the driving part is disposed inside the first region and is mechanically coupled to the driving part at the coupling portion formed on the inner wall surface of the first region. The shape of the first region is not limited to a particular shape so long as such a state of use can be achieved, and the first region may or may not penetrate the member body.

In the present embodiment, the driving part may include a drive source that contains predetermined energy for generating the pressing force, and may be configured to generate the pressing force by releasing the predetermined energy to outside. The drive source may be an ignition charge, and the driving part may be configured to generate the pressing force by releasing combustion energy which is caused by combustion of the ignition charge and is the predetermined energy. In addition, the driving part may utilize electrical energy of a piezoelectric element or the like or mechanical energy of a spring or the like as the pressing force instead of the pressing force caused by the combustion of the ignition charge, and may generate the pressing force by appropriately combining these forms of energy.

Here, the driving part may be configured to complete the release of the predetermined energy within 10 msec from start of actuation of the driving part. That is, an embodiment in which as a large predetermined energy is released in a very short period of time, a large impact load is applied to the coupling portion of the crack resistant member in a very short period of time can be given. In this case, as the drive source, it is preferable to use an ignition charge, for example, any one of an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), and an explosive containing aluminum and iron oxide (AFO), or an explosive composed of a plurality of these explosives in combination. The reason for this is as follows. In these ignition charges, the combustion product is a gas at a high temperature but does not include a gas component at room temperature, hence the combustion product is condensed immediately after ignition. As a result, the driving part releases the combustion energy in an extremely short period of time.

In the crack resistant member according to the present embodiment, the coupling portion may be a screw portion formed on the inner wall surface of the first region along the extension direction of the first region. That is, the driving part has such a cylindrical shape that fills the first region, and the screw portion is formed on an outer wall of the driving part. When the screw portion formed on the outer wall of the driving part is screwed with the screw portion formed on the inner wall of the first region, the crack resistant member and the driving part can be firmly fixed. However, as long as the crack resistant member and the driving part can be coupled mechanically, the coupling portion may be configured to mechanically couple with the driving part by a coupling method other than screwing. Examples of coupling methods other than screwing include snap fitting, riveting, and the like. Before or after the driving part is disposed inside the first region, the resin material for forming the coupling portion may be heated and melted and further solidified by cooling, so that coupling strength may be improved.

In the crack resistant member according to the present embodiment, although at least the coupling portion is formed from a resin material having a flexural modulus of 2600 MPa or greater as measured in accordance with ISO 178, other portions may be formed from the resin material, or may be formed from a material other than the resin material. However, from the viewpoint of improving crack resistance, the entire crack resistant member is preferably integrally formed from the resin material.

The lower limit of the flexural modulus of the resin material is preferably 2600 MPa or greater, more preferably 2640 MPa or greater, even more preferably 2680 MPa or greater, and particularly preferably 2720 MPa or greater. The upper limit of the flexural modulus of the resin material is normally 4000 MPa or less, preferably 3900 MPa or less, more preferably 3800 MPa or less, even more preferably 3700 MPa or less, and particularly preferably 3600 MPa or less.

With the flexural modulus of the resin material in the range described above, even when the impact load is applied to the coupling portion, it is possible to suppress crack generation and crack growth at the coupling portion and/or near the coupling portion.

The flexural modulus of the resin material is measured by a bending tester (for example, a universal material testing machine 5966 available from Instron Corp) in accordance with ISO 178 after a test piece having dimensions of 80 mm × 10 mm × 4 mm produced by injection molding is left to stand for 24 hours or longer in an environment at 23°C and 50% RH. However, when a commercially available product is used as the resin material, a catalog value can be employed.

In the crack resistant member according to the present embodiment, the resin material preferably contains one or more selected from the group consisting of a mixture of a polycarbonate resin and an acrylonitrile-butadiene-styrene copolymer (ABS resin), a polyolefin-based resin, a crystalline polyamide resin, and a crystalline polyester resin. This is because the flexural modulus of these resins is high, and thus it is possible to easily adjust the flexural modulus of the resin material to a desired value without compounding a reinforcing agent such as glass fiber, alumina, or silica.

The content of these resins relative to a total amount of the resin material is not particularly limited, but is usually 70 mass% or greater, preferably 80 mass% or greater, more preferably 85% or greater, even more preferably 90 mass% or greater, particularly preferably 95 mass% or greater, and usually 100 mass% or less.

The mixture of the polycarbonate resin and the ABS resin contains only polycarbonate resin and ABS resin.

A mixing ratio of both resins in the mixture of the polycarbonate resin and the ABS resin is not particularly limited, and a proportion of the ABS resin (hereinafter referred to as "ABS resin content") with respect to a total amount of the polycarbonate resin and the ABS resin is usually 5 mass% or greater, preferably 10 mass% or greater, more preferably 14 mass% or greater, and even more preferably 18 mass% or greater, and is usually 35 mass% or less, preferably 30 mass% or less, more preferably 26 mass% or less, and even more preferably 22 mass% or less.

With the ABS resin content in the range described above, it becomes easy to prepare a resin material having a flexural modulus of 2600 MPa or greater, or further 2700 MPa or greater.

The polycarbonate resin is not particularly limited as long as it is a polymer having a carbonate bond (-OCO₂-) in the main chain, and a well-known resin or a resin that can be easily produced by a method according to a known production method can be used. The polycarbonate resin is generally obtained by an interface condensation polymerization reaction between a polyhydric alcohol component and a halogenated carbonyl component or by a transesterification reaction between a polyhydric alcohol component and a carbonic diester component.

Examples of the polyhydric alcohol component include aliphatic diols such as ethylene glycol, 1,2-propanediol, 1,3-propanediol, 2,2-dimethylpropane-1,3-diol, 2-methyl-2-propyl propane-1,3-diol, 1,4-butanediol, 1,6-hexanediol, 1,2-cyclopentanediol, 1,2-cyclohexanediol, 1,4-cyclohexanediol, 1,4-cyclohexanedimethanol, 4-(2-hydroxyethyl)cyclohexanol, 2,2,4,4-tetramethyl-1,3-cyclobutanediol; aromatic diols such as 1,2-dihydroxybenzene, 1,3-dihydroxybenzene, 1,4-dihydroxybenzene, 2,2'-dihydroxy-1,1'-binaphthyl, 1,2-dihydroxynaphthalene, 1,3-dihydroxynaphthalene, 2,3-dihydroxynaphthalene, 1,5-dihydroxynaphthalene, 2,6-dihydroxynaphthalene, 1,7-dihydroxynaphthalene, 2,7-dihydroxynaphthalene, bis(2-hydroxyphenyl)ether, bis(3-hydroxyphenyl)ether, bis(4-hydroxyphenyl)ether, 1,4-bis(3-hydroxyphenoxy)benzene, 1,3-bis(4-hydroxyphenoxy)benzene, 2,2-bis (4-hydroxyphenyl)propane (i.e., bisphenol A), 1,1-bis(4-hydroxyphenyl)propane, 2,2-bis(4-hydroxy-3-methylphenyl)propane, 2,2-bis(3-methoxy-4-hydroxyphenyl)propane, 2-(4-hydroxyphenyl)-2-(3-methoxy-4-hydroxyphenyl)propane, 1,1-bis(3-tert-butyl-4-hydroxyphenyl)propane, 2,2-bis(3,5-dimethyl-4-hydroxyphenyl)propane, 2,2-bis(3-cyclohexyl-4-hydroxyphenyl)propane, 2-(4-hydroxyphenyl)-2-(3-cyclohexyl-4-hydroxyphenyl)propane, a,a'-bis(4-hydroxyphenyl)-1,4-diisopropylbenzene, 1,3-bis[2-(4-hydroxyphenyl)-2-propyl]benzene, bis(4-hydroxyphenyl)methane, 1,1-bis(4-hydroxyphenyl)cyclohexylmethane, bis(4-hydroxyphenyl)phenylmethane, bis(4-hydroxyphenyl) (4-propenylphenyl)methane, bis(4-hydroxyphenyl)diphenylmethane, bis(4-hydroxyphenyl)naphthylmethane; and the like.

Examples of the halogenated carbonyl component include carbonyl chloride (phosgene), carbonyl bromide, and carbonyl iodide.

Examples of the carbonic diester component include dialkyl carbonates such as dimethyl carbonate, diethyl carbonate, di-tert-butyl carbonate, and dicyclohexyl carbonate; diaryl carbonates such as diphenylcarbonate, ditolyl carbonate, phenyltolyl carbonate, di-p-chlorophenyl carbonate, and dinaphthyl carbonate; alkylaryl carbonates such as methylphenylcarbonate, ethylphenyl carbonate, and cyclohexylphenyl carbonate; dialkenyl carbonates such as divinyl carbonate, diisopropenyl carbonate, and dipropenyl carbonate; and the like.

A viscosity average molecular weight (Mv) of the polycarbonate resin is not particularly limited, but from the viewpoint of crack resistance, the lower limit is preferably 10000 or greater and more preferably 20000 or greater, and the upper limit is preferably 27000 or less and more preferably 35000 or less.

The ABS resin is not particularly limited, and a well-known resin or a resin that can be easily produced by a method according to a known production method can be used. Examples thereof include a resin having a monomer configuration from 10 to 20 mass% of acrylonitrile, from 40 to 70 mass% of butadiene, and from 20 to 40 mass% of styrene, a resin obtained by substituting styrene and/or acrylonitrile in ABS resin with other monomers, a resin obtained by substituting a rubber component mainly composed of butadiene of ABS resin with other rubber components, and the like. Such an ABS resin can be produced by a known production method or a method according to a known production method. Examples of known methods include emulsion polymerization, solution polymerization, suspension polymerization, bulk polymerization, and the like. The polymerization may be carried out in one step or in multiple steps.

Commercially available products can be used as the mixture of the polycarbonate resin and the ABS resin. Examples of commercially available products include NOVALLOY S69P1, NOVALLOY S6700, and NOVALLOY S6500L (all available from Daicel Polymer Ltd.).

The polyolefin-based resin is not particularly limited, and a well-known resin or a resin that can be easily produced by a method according to a known production method can be used. Examples thereof include homopolymers of olefins or copolymers of two or more olefins, random or block copolymers of one or more olefins and one or more polymerizable monomers that are polymerizable with the olefin, and modified products of these polymers. The polyolefin-based resin may be a mixture of two or more types of different polyolefin-based resins.

Examples of the olefin constituting the polyolefin-based resin include α-olefins having from 2 to 20 carbon atoms, cyclic olefins, and the like.

Examples of α-olefins having from 2 to 20 carbon atoms include ethylene, propylene, 1-butene, 2-methyl-1-propene, 1-pentene, 2-methyl-1-butene, 3-methyl-1-butene, 1-hexene, 2-ethyl-1-butene, 2,3-dimethyl-1-butene, 2-methyl-1-pentene, 3-methyl-1-pentene, 4-methyl-1-pentene, 3,3-dimethyl-1-butene, 1-heptene, 2-methyl-1-hexene, 2,3-dimethyl-1-pentene, 2-ethyl-1-pentene, 2-methyl-3-ethyl-1-butene, 1-octene, 2-ethyl-1-hexene, 3,3-dimethyl-1-hexene, 2-propyl-1-heptene, 2-methyl-3-ethyl-1-heptene, 2,3,4-trimethyl-1-pentene, 2-propyl-1-pentene, 2,3-diethyl-1-butene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, and the like.

Examples of cyclic olefins include norbonne derivatives such as bicyclo[2.2.1]hept-2-ene, 6-alkylbicyclo[2.2.1]hept-2-ene, 5,6-dialkylbicyclo[2.2.1]hept-2-ene, 1-alkylbicyclo[2.2.1]hept-2-ene, and 7-alkylbicyclo[2.2.1]hept-2-ene; dimethanooctahydronaphthalene derivatives such as tetracyclo[4.4.0.1^{2,5}.1^{7,10}]-3-dodecene, 8-alkyltetracyclo[4.4.0.1^{2,5}. 1^{7,10}]-3-dodecene, and 8,9-dialkyltetracyclo[4.4.0.1^{2,5}.1^{7,1}]-3-dodecene; and the like.

Examples of one or more polymerizable monomers that are polymerizable with an olefin include unsaturated organic acids or derivatives thereof such as acrylic acid, methacrylic acid, maleic acid, itaconic acid, methyl acrylate, ethyl acrylate, methyl methacrylate, maleic anhydride, glycidyl methacrylic acid, aryl maleic imide, and alkyl maleic imide; vinyl esters such as vinyl acetate and vinyl butyrate; aromatic vinyl compounds such as styrene and methyl styrene; vinyl silanes such as vinyltrimethylmethoxysilane, and methacryloyloxypropyltrimethoxysilane; non-conjugated dienes such as dicyclopentadiene and 4-ethylidene-2-norbornene; and the like.

Specific examples of polyolefin-based resins include low density polyethylene, linear low density polyethylene (ethylene-α-olefin copolymer), high density polyethylene, polypropylene, propylene-ethylene copolymer, propylene-1-butene copolymer copolymer, ethylene-cyclic olefin copolymer, ethylene-vinylcyclohexane copolymers, poly(4-methylpentene-1), poly(butene-1), ethylene-methyl acrylate copolymers, ethylene-methyl methacrylate copolymers, ethylene-ethyl acrylate copolymers, ethylene-vinyl acetate copolymers, and the like. Of these, low density polyethylene, linear low density polyethylene, high density polyethylene, or polypropylene are preferable, and polypropylene is more preferable.

The crystalline polyester resin is not particularly limited as long as it is a polymer having an ester bond (-CO₂-) in the main chain and crystalline, and a well-known resin or a resin that can be easily produced by a method according to a known production method can be used. The crystalline polyester resin is generally obtained by polymerizing a polybasic carboxylic acid component and a polyhydric alcohol component, a hydroxycarboxylic acid component, and/or a lactone component.

Examples of the polyvalent carboxylic acid component include aliphatic dicarboxylic acids such as oxalic acid, succinic acid, glutaric acid, adipic acid, speric acid, azelaic acid, sebacic acid, 1,9-nonandicarboxylic acid, 1,10-decanedicarboxylic acid, 1,12-dodecanedicarboxylic acid, 1,14-tetradecanedicarboxylic acid, and 1,18-octadecanedicarboxylic acid; aromatic dicarboxylic acids such as phthalic acid, isophthalic acid, terephthalic acid, naphthalene-2,6-dicarboxylic acid, and malonic acid; trivalent or higher aliphatic polycarboxylic acids such as 1,2,4-butanetricarboxylic acid and 1,2,5-hexanetricarboxylic acid; trivalent or higher aromatic polycarboxylic acids such as trimellitic acid and pyromellitic acid; derivatives thereof such as anhydrides and alkyl (having from 1 to 3 carbon atoms) esters thereof; and the like.

Examples of the polyhydric alcohol component include aliphatic diols such as ethylene glycol, 1,3-propanediol, 1,2-propanediol, 1,3-butanediol, 2,3-butanediol, 1,4-butanediol, diethylene glycol, 2-methyl-1,3-propanediol, triethylene glycol, neopentyl glycol, 1,6-hexanediol, and 1,8-octanediol; cycloaliphatic diols such as isosorbide, isomannide, 1,2-cyclohexanedimethanol, 1,4-cyclohexanedimethanol, and 1,4-cyclohexanediol; aromatic diols such as hydroquinone, 4,4'-hydroxybisphenol, 1,4-bis(β-hydroxyethoxy)benzene, 1,4-bis(P-hydroxyethoxyphenyl)sulfone, bis(p-hydroxyphenyl)ether, bis(p-hydroxyphenyl)sulfone, bis(p-hydroxyphenyl)methane, 1,2-bis (p-hydroxyphenyl)ethane, bisphenol A, and alkylene oxide adduct of bisphenol A; trivalent or higher alcohols such as sorbitol, 1,2,3,6-hexane tetralol, 1,4-sorbitan, pentaerythritol, dipentaerythritol, 1,2,4-butanetriol, 1,2,5-pentanetriol, glycerol, 2-methylpropanetriol, 2-methyl-1,2,4-butanetriol, trimethylolethane, trimethylolpropane, and mannitol; and the like.

Examples of the hydroxycarboxylic acid component include α-hydroxycarboxylic acids such as glycolic acid, lactic acid, α-hydroxybutyric acid, α-hydroxyisobutyric acid, α-hydroxyvaleric acid, α-hydroxycaproic acid, α-hydroxyisocaproic acid, α-hydroxyheptanoic acid, α-hydroxyoctanoic acid, α-hydroxydecanoic acid, α-hydroxymyristic acid, α-hydroxystearic acid, mandelic acid, malic acid, and citric acid; β-hydroxycarboxylic acids such as β-lactic acid, β-hydroxybutyric acid, β-hydroxyvaleric acid, β-hydroxyisovaleric acid, and salicylic acid; γ-hydroxycarboxylic acids such as γ-hydroxybutyric acid and γ-hydroxyvaleric acid; and the like.

Examples of the lactone component include β-propiolactone, ε-propiolactone, β-butyrolactone, γ-butyrolactone, pivalolactone, δ-valerolactone, β-methyl-δ-valerolactone, ε-caprolactone, dodecanolactone, and the like.

Specific examples of the crystalline polyester resin include polyethylene terephthalate, polybutylene terephthalate, polypropylene terephthalate, polyhexamethylene terephthalate, polycyclohexylene dimethylene terephthalate, polybutylene naphthalate, polyethylene naphthalate, and the like.

The crystalline polyamide resin is not particularly limited as long as it is a polymer having an amide bond (-CONH-) in the main chain and crystalline, and a well-known resin or a resin that can be easily produced by a method according to a known production method can be used. The crystalline polyester resin is generally obtained by polymerizing a polybasic carboxylic acid component and a polybasic amine component, an α,ω-amino acid component, and/or a lactam.

Examples of the polybasic carboxylic acid component include aliphatic dicarboxylic acids such as oxalic acid, malonic acid, succinic acid, glutaric acid, adipic acid, pimelic acid, suberic acid, azelaic acid, sebacic acid, undecanedioic acid, dodecanedioic acid, tridecanedioic acid, tetradecanedioic acid, pentadecanedioic acid, and hexadecanedioic acid; alicyclic dicarboxylic acids such as 1,4-cyclohexanedicarboxylic acid, 1,3-cyclohexanedicarboxylic acid, 1,2-cyclohexanedicarboxylic acid, and 1,3-cyclopentanedicarboxylic acid; aromatic dicarboxylic acids such as phthalic acid, isophthalic acid, terephthalic acid, 2,6-naphthalene dicarboxylic acid, 1,5-naphthalene dicarboxylic acid, 4,4'-diphenyldicarboxylic acid, bis(p-carboxyphenyl)methane, anthracenedicarboxylic acid, 4,4'-diphenyletherdicarboxylic acid, and 5-sodium sulfoisophthalic acid; derivatives thereof such as anhydrides and alkyl (from 1 to 3 carbon atoms) esters thereof; and the like.

Examples of the polybasic amine component include aliphatic diamines such as ethylene diamine, 1,3-diaminopropane, 1,4-diaminobutane, 1,6-diaminohexane, 1,7-diaminoheptane, 1,8-diaminooctane, 1,9-diaminononane, 1,10-diaminodecane, 1,11-diaminodecane, 1,12-diaminododecane, 1,14-diaminotetradecane, 1,16-diaminohexadecane, 1,18-diaminooctadecane, and 1,20-diaminoeicosane; alicyclic diamines such as cyclohexane diamine, isophorone diamine, norbornandimethylamine, 4,4'-diaminodicyclohexylmethane, 2,2-(4,4'-diaminodicyclohexyl)propane, and 3,3'-dimetyl-4,4'-diaminohexylethane; aromatic diamines such as m-xylylene diamine; and the like.

Examples of the α,ω-amino acid component include 1,4-aminobutanoic acid, 1,6-aminohexanoic acid, 1,7-diaminoheptanoic acid, 1,8-aminooctanoic acid, 1,11-aminoundecanoic acid, 1,12-aminododecanoic acid, 4-(aminomethyl) benzoic acid, and the like.

Examples of the lactam component include pyrrolidin-2-one, ε-caprolactam, ω-laurolactam, enantholactam, capryllactam, laurinlactam, and the like.

Specific examples of the crystalline polyamide resin include polycaprolactam (nylon 6), polytetramethylenadipamide (nylon 46), polyhexamethylenadipamide (nylon 66), polyhexamethylenesebacamide (nylon 610), polyhexamethylenedodecamide (nylon 612), polyundecamethylene adipamide (nylon 116), polyundecalactum (nylon 11), polydodecalactum (nylon 12), polytrimethylhexamethyleneterephthalamide (nylon TMHT), polyhexamethylene isophthalamide (nylon 61), polynonamethylene terephthalamide (nylon 9T), polyhexamethylene terephthalamide (nylon 6T), polybis(3-methyl-4-aminohexyl)methandodecamide (nylon PACM12), polybis(3-methyl-4-aminohexyl)methane tetradecamide (nylon PACM14), polymetaxylylene adipamide (nylon MXD6), polyundecamethylene terephthalamide (nylon 11T), polyundecamethylene hexahydroterephthalamide (nylon 11T (H)), and the like.

In the present embodiment, the resin material may include optional components within a range that does not impair the effects of the present disclosure. Examples of the optional components include known additives such as impact modifiers, crystal nucleating agents, inorganic and organic antimicrobial agents, stabilizers, flame retardants, flame retardant adjuvants, anti-dripping agents, plasticizers, flow modifiers, antioxidants, lubricants, release agents, light stabilizers, weather resistant agents, colorants, pigments, fluorescent brightening agents, modifiers, antistatic agents, anti-hydrolysis agents, fillers, reinforcing fibers, reinforcing agents other than reinforcing fibers, and the like.

In the present embodiment, the method of producing the resin material is not particularly limited, and the resin material can be produced by a melt-kneading method for a resin component and optional components added as necessary.

The melt-kneading method may be performed by a known method. Examples of known methods include a method in which the resin component and optional components contained as necessary are mixed by a mixing device such as a Henschel mixer, a ribbon blender, a V blender, or a tumble mixer, and then the mixture is melt-kneaded using a kneader such as a single or multi-screw kneading extruder, a calender, a Banbury mixer, or a kneading roll; a method in which the resin component and optional components contained as necessary are continuously supplied at a constant ratio using a fixed-quantity supply machine to obtain a mixture, and the mixture is then melt-kneaded using a kneader such as a single or multi-screw kneading extruder, a calender, a Banbury mixer, or a kneading roll; and other methods.

Although the temperature and kneading time for melt-kneading can be selected according to the type of resin component, the proportion of the components, the type of kneader, and the like, the temperature for melt-kneading is preferably in a range of 250°C or higher and 320°C or lower. With the temperature in the above range, thermal deterioration of the resin component, reduction in physical properties of the crack resistant member, and deterioration in appearance of the crack resistant member can be suppressed.

When the resin material is made of only a resin component and a mixture of one type of resin component or two or more types of resin components is available in the form of pellets, powders, granules, and the like, the resin component or the mixture of resin components can be used as is as a resin material.

A method of producing, from the resin material, at least the coupling portion of the crack resistant member, preferably the entire crack resistant member is not particularly limited, and they can be produced by a known molding method. Examples of known molding methods include injection molding, insert molding, blow molding, extrusion molding, compression molding, and the like. Of these, injection molding is preferable from the viewpoint of crack resistance and moldability.

The resin temperature during molding is preferably higher than or equal to a melting point of the resin component (resin component having a higher melting point when the resin material contains two or more resin components) in the resin material, and is preferably lower than or equal to the melting point plus 320°C. With the temperature range described above, sufficient melt viscosity and fluidity can be obtained, high moldability can be ensured, and coloration of the resin can be suppressed.

The crack resistant member according to the present embodiment can be used in various applications as long as it is used in the use state described above, and can be suitably used as an attachment for a needleless injector described below. Among these, the crack resistant member can be particularly suitably used as an attachment for a needleless injector that ejects an intended injection substance by injection energy from combustion of an ignition charge.

### <Needleless injector>

A needleless injector according to another embodiment of the present disclosure is a needleless injector configured to eject an intended injection substance to a target region without using an injection needle, and includes an attachment that is the crack resistant member described above and couples an actuator, which is the driving part, into the attachment with the coupling portion; the actuator; a housing part that has an accommodating space that accommodates the intended injection substance and defines a flow path from the accommodating space in such a manner that the intended injection substance is ejected to the target region, a metal piston which is arranged to propel inside the needleless injector in a predetermined direction when the pressing force is applied by actuation of the driving part, and a plunger that defines the accommodating space and propels inside the housing part by the pressing force to pressurize the intended injection substance.

In the needleless injector according to the present embodiment, the driving part imparts the pressing force to the intended injection substance accommodated in the housing part, and thus the intended injection substance is ejected to the target region. In the present application, "ejection" is achieved by the plunger propelling in the housing part through the pressing force imparted by the driving part and thus pressurizing the intended injection substance in the housing part, so that the intended injection substance flows through the flow path in the housing part.

Further, as the intended injection substance ejected from the needleless injector, predetermined substances including a component expected to have effects in the target region or a component expected to exert a predetermined function in the target region can be exemplified. Thus, as long as at least ejection by the pressing force described above can be achieved, a physical form of the intended injection substance may be in a state of being dissolved in liquid, or may be in a state of being simply mixed without being dissolved in liquid. As one example, the predetermined substance to be sent includes vaccine for intensifying an antibody, a protein for cosmetic enhancement, a cultured cell for hair regeneration, and the like, and is included in a liquid medium in an ejectable manner. The intended injection substance is formed in this way. Note that the medium is preferably a medium that does not hinder the above-mentioned effect and function of the predetermined substance in a state of being injected into the target region. As another method, the medium may be a medium that exerts the above-mentioned effect and function by acting together with the predetermined substance in the state of being injected into the target region.

The intended injection substance ejected needs to penetrate the surface of the target region such that the intended injection substance is ejected from the needleless injector to the target region to be delivered into the inside thereof. Thus, at an ejection initial state, the intended injection substance needs to be ejected to the target region at a relatively high speed. In view of this point, as an example, the driving part preferably contains the above-mentioned ignition charge as a drive source containing combustion energy and imparts the pressing force in an extremely short period of time by using a combustion product discharged by combustion of the ignition charge.

Here, the metal piston is arranged to propels inside the needleless injector in a predetermined direction when the pressing force is applied. The plunger is disposed adjacent to the piston and is configured to pressurize the intended injection substance in the housing part through the pressing force imparted by the driving part. Thus, during the pressurization by the plunger, relatively large force can be applied to the housing part itself constituting the needleless injector. In particular, due to the pressurization by the plunger, the intended injection substance will be pushed into the flow path provided in the housing part, so that the housing part will be subjected to force directly from the plunger or through the intended injection substance until the plunger that is propelled in the housing part toward the deepest part is in direct contact with an inner wall surface of the deepest part.

With reference to the drawings, a needleless injector 1 according to an embodiment of the present disclosure (herein, simply referred to as "injector") is described below. The injector 1 is a needleless injector that implements injection by ejecting an ejection solution, which corresponds to an intended injection substance in the present application, to a target region through use of a combustion energy of an ignition charge, that is, a device that injects the ejection solution to the target region without using an injection needle.

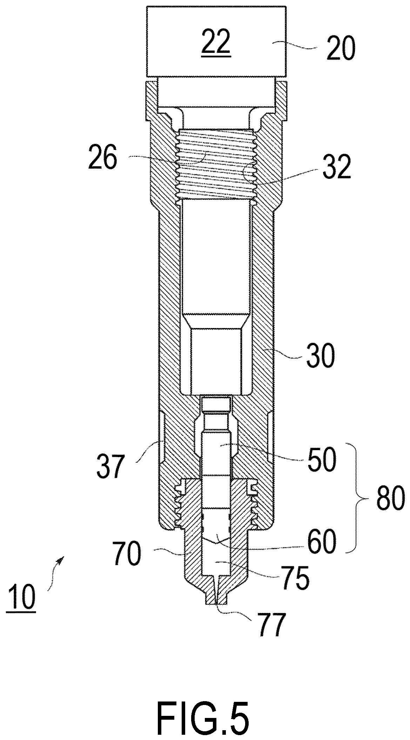

Note that, configurations of the following embodiment are provided as examples, and the present disclosure is not limited to the configurations of the embodiment. Note that, in the present embodiment, as terms indicating a relative positional relationship in a longitudinal direction of the injector 1, "distal end side" and "base end side" are used. The "distal end side" indicates a side closer to the distal end of the injector 1 described later, that is, a position closer to an ejection port 77, and the "base end side" indicates a side in an opposite direction to the "distal end side" in a longitudinal direction of the injector 1, that is, a direction to an igniter 22 side of an injector assembly 10 (see FIG. 5 described later).

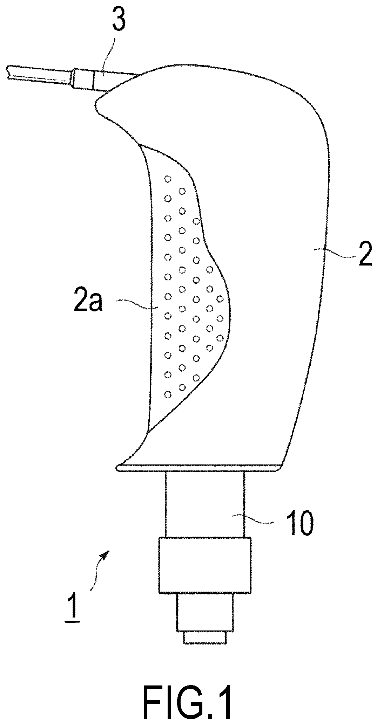

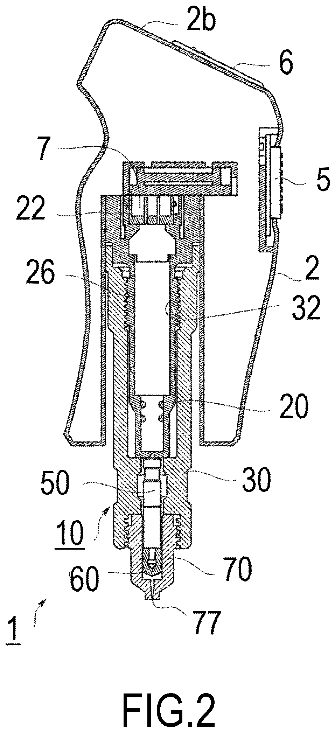

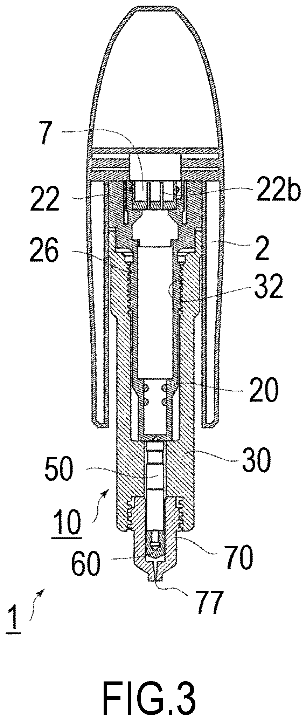

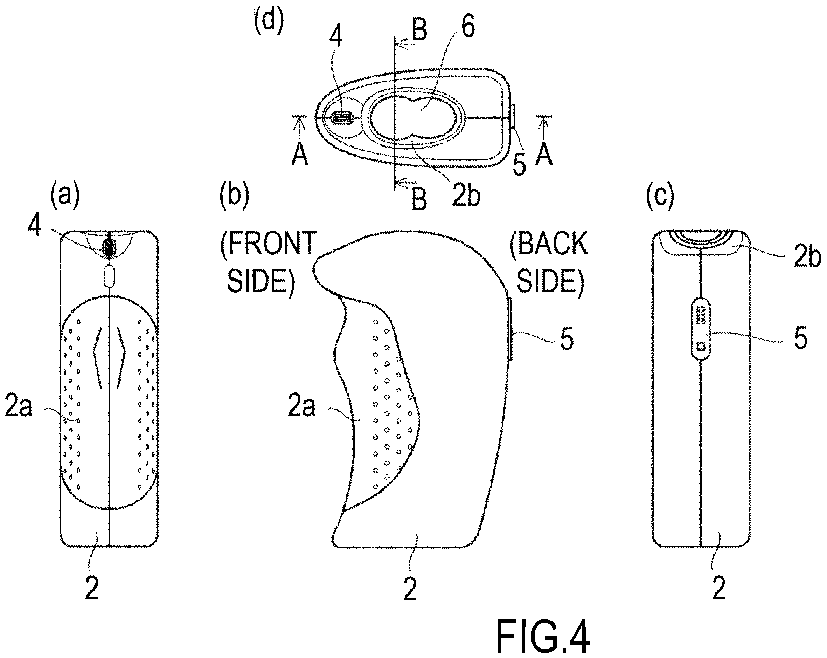

Here, FIG. 1 is a diagram schematically illustrating the appearance of the injector 1. FIG. 2 is a first cross-sectional view of the injector 1, which is an AA cross section in FIG. 4, described below. FIG. 3 is a second cross-sectional view of injector 1, a BB cross section in FIG. 4 described below. The BB cross section is orthogonal to the AA cross section. Note that FIG. 4 is a diagram illustrating a configuration of a housing 2 that is a part of the injector 1. Here, the injector 1 is formed with the injector assembly 10 attached to the housing 2. A power cable 3 for supplying drive current to the igniter 22 in the injector assembly 10 is connected to the housing 2.

Note that, in the following description in the present application, the ejection solution ejected to the target region by the injector 1 is formed of a liquid medium including a predetermined substance, which exerts an effect or a function expected in the target region. In the ejection solution, the predetermined substance may be in a state of being dissolved in liquid being a medium, or may be in a state of being simply mixed instead of being dissolved.

For example, examples of the predetermined substance included in the ejection solution include an organism-derived substance and a substance with a desired bioactivity, which can be ejected to the target region being an organism. For example, examples of the organism-derived substance include DNA, RNA, a nucleic acid, an antibody, and a cell. Examples of the substance with a desired bioactivity include various substances exerting pharmacological or therapeutic effects, which are exemplified by medicines composed of low molecular compounds, proteins, peptides, or the like, a vaccine, an inorganic substance such as metal particles for thermotherapy or radiotherapy, and a carrying body functioning as a carrier. Further, the liquid being the medium of the ejection solution is only required to be a substance suitable for administering the predetermined substance exemplified by those substances to the target region, and may be aqueous or oleaginous, which is not limited. Further, viscosity of the liquid being the medium is not particularly limited as long as the predetermined substance can be ejected by the injector 1.

In the injector 1, the injector assembly 10 is configured to be attachable to and detachable from the housing 2 freely. An accommodating space 75 (see FIG. 5) formed between a container 70 and a plunger 80 in the injector assembly 10 is filled with ejection solution during a preparation stage before the operation of the injector 1. The injector assembly 10 is a unit that is replaced each time the ejection solution is ejected. The injector assembly 10 will be described in detail below.

On the other hand, the housing 2 has a grip portion 2a formed to be gripped by a user of the injector 1 in use, and is provided with a plurality of switches for operating the injector 1 to eject the ejection solution. Note that the injector 1 is configured to be capable of being held and operated by one hand of the user. In this context, the housing 2 will be described with reference to FIG. 4. In FIG. 4, (a) illustrates the outer appearance of the housing 2 as viewed from the front side, (b) illustrates the outer appearance of the housing 2 as viewed from one side, (c) illustrates the outer appearance of the housing 2 as viewed from the back side, and (d) illustrates the outer appearance of the housing 2 as viewed from the upper side. Here, "front side" indicates a portion positioned on the distal side of the user holding the housing 2, which is the left side in FIG. 4(b), and "back side" indicates a portion positioned on the proximal side of the user holding the housing 2, which is the right side in FIG. 4(b). Thus, when the user holds the housing 2 with one hand, fingertips rest on the front side of the distal housing 2 which is the distal side, and the wrist is in the vicinity of the back side of the housing 2 which is the proximal side. The "upper side" is a portion of the injector 1 on the base end side.

Considering such a way of holding by the user, the grip portion 2a is provided at a front side portion of the housing 2 so that the user can easily rest his or her fingertips thereon. The grip portion 2a is provided with a plurality of dimples making the user's fingertips even easier to be rested thereon. Furthermore, the grip portion 2a has gentle recesses and protrusions on the front side of its outer shell (see (b) in FIG. 4) so that the user's forefinger and middle finger can be easily rested thereon, for the sake of more stable holding of the housing by the user.

Further, the housing 2 is provided with a first switch 5 and a second switch 6 that are two operating switches for operating the injector 1. A first switch 5 and a second switch 6 are connected to a control unit, such as a microcomputer (not illustrated). The control unit controls the supply of ignition current to the igniter 22 based on a signal from each switch, thereby controlling an operation of the injector 1. The first switch 5 is a sliding switch provided on the back side of the housing 2, the sliding direction of which being an upward and downward direction of the housing 2 (direction between the distal end and the base end). The first switch 5 is constantly biased in the upward direction. The user can achieve a standby state of the injector 1 by continuously sliding the first switch 5 downward (toward the distal end side) for a predetermined period of time against the biasing force. The standby state is a state in which the injector 1 is ready to eject the ejection solution. When a user makes an additional operation in this state, the ejection is implemented.

The second switch 6 is a press type switch provided on an inclined surface 2b on the upper side of the housing 2. The user can press the second switch 6 in a direction toward the inner side of the housing 2. The control unit is configured to supply an ignition current to the igniter 22 in response to the pressing operation on the second switch 6 while the injector 1 is in the standby state as a result of the operation on the first switch 5 described above. A connector 4 to which the power cable 3 is connected is provided on the front side of the inclined surface 2b on the upper side of the housing 2. In the present embodiment, the connector 4 is a USB connector, and the power cable 3 is freely attachable to and detachable the housing 2.

Note that, as described above, in the present embodiment, the power for actuating the igniter 22 is supplied from the outside through the power cable 3. Alternatively, a battery for supplying such power may be provided inside the housing 2. In this case, the housing 2 can be repeatedly used while replacing the injector assembly 10, until the battery runs out. When the battery runs out, the battery may be replaced.

A schematic configuration of the injector assembly 10 is illustrated in FIG. 5. The injector assembly 10 is attached to the housing 2 to form the injector 1, as illustrated in FIGS. 2 and 3. Specifically, the injector assembly 10 is an assembly including an actuator 20, an attachment 30, the container 70, and the plunger 80. How the injector assembly 10 is assembled will be described below.

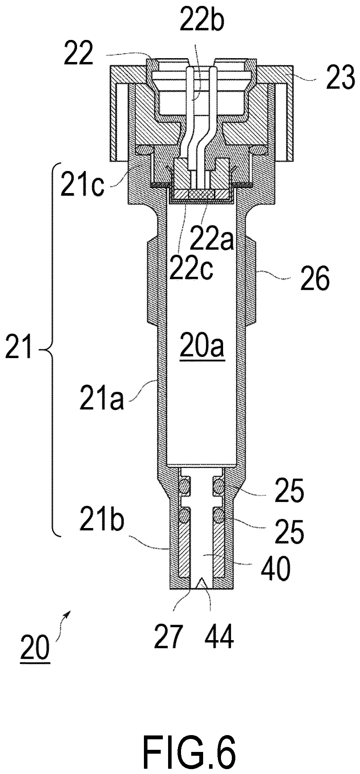

First of all, the actuator 20 will be described with reference to FIG. 6. The actuator 20 has a body 21 formed in a cylindrical shape. The body 21 includes a center portion 21a in the center thereof, a distal end portion 21b on the distal end side thereof, and a base end portion 21c on the base end side thereof. The distal end portion 21b, the center portion 21a, and the base end portion 21c of the body 21 have their internal spaces in communication with each other. The distal end portion 21b has an opening 27 on the distal end side. The igniter 22, which is an electric igniter that generates energy for ejection through combustion of an ignition charge 22a, is attached to the base end portion 21c of the body 21 via a cap 23. The igniter 22 has an ignition pin 22b to which ignition current is supplied from the outside. The ignition pin 22b is coupled to a socket 7 on the side of the housing 2 in a state in which the injector assembly 10 is attached to the housing 2. The attachment state of the igniter 22 to the body 21 is determined such that a combustion product generated by the operation of the igniter 22 is discharged toward the center portion 21a of the body 21. Specifically, the igniter 22 is attached to the base end portion 21c of the body 21 to have a discharge surface 22c, from which the combustion product is discharged, directed toward the center portion 21a.

Herein, a combustion energy used in the igniter 22 for the ignition charge is an energy for the injector 1 to eject the ejection solution to the target region. Note that, examples of the ignition charge include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), or an explosive composed of a combination of a plurality of these explosives. These explosives exhibit characteristics that, although the explosives generate high-temperature and highpressure plasma during combustion immediately after ignition, when the combustion product condenses at room temperature, the explosives do not contain gaseous components and hence the pressure generated decreases abruptly. An explosive other than these may be used as the ignition charge as long as appropriate ejection of the ejection solution can be performed.

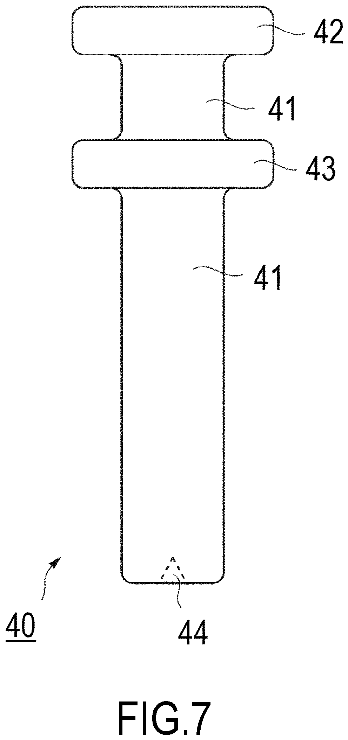

The internal space of the center portion 21a of the body 21 serves as a combustion chamber 20a into which a combustion product is discharged from the igniter 22. Furthermore, a male thread portion 26 is formed in a part of the outer surface of the center portion 21a. The male thread portion 26 is configured to mate with a female thread portion 32 of the attachment 30 described below. The effective lengths of the male thread portion 26 and the female thread portion 32 are determined to guarantee sufficient coupling force therebetween. The internal space of the distal end portion 21b adjacent to the center portion 21a is formed in a cylindrical shape in which a piston 40 is slidably provided and O rings 25 serving as a sealing member are also provided. The piston 40 is made of metal, has a shaft member 41, is provided with a first flange 42 on the base end side thereof, and is further provided with a second flange 43 in the vicinity of the first flange 42, as illustrated in FIG. 7. The first flange 42 and the second flange 43 have a disc shape, and have the same diameter. The O rings 25 include one disposed between the first flange 42 and the second flange 43 and one disposed on another side of the second flange 43. A recess portion 44 having a predetermined size is formed in a distal end surface of the shaft member 41. In a state where the piston 40 is disposed in the internal space of the distal end portion 21b before the actuation of the actuator 20, the first flange 42, which serves as a surface receiving pressure of the combustion product from the igniter 22, is exposed on side of the combustion chamber 20a, and the distal end of the shaft member 41 of the piston 40 is inserted into the opening 27.

Then, when the igniter 22 is activated and the combustion product is discharged into the combustion chamber 20a and thus the pressure therein rises, the first flange 42 receives the pressure, resulting in the piston 40 sliding toward the distal end side. Thus, the actuator 20 has a mechanism with the igniter 22 serving as an actuation source and the piston 40 serving as an output unit. Since the second flange 43 has a larger diameter than the opening 27, the distance by which the piston 40 can slide is limited. Thus, the distance by which the shaft member 41 of the piston 40 can protrude from the distal end surface of the distal end portion 21b of the body 21 is limited. Further, the piston 40 may be formed of a resin, and in such case, metal may be used together for a part to which heat resistance and pressure resistance are required.

Additionally, as an alternative mechanism to adjust the pressure applied to the piston 40, the combustion chamber 20a of the actuator 20 may be further provided with a gas generating agent that is burned by the combustion product from the igniter 22 to produce gas. The agent may be disposed, for example, at a location that may be exposed to the combustion product from the igniter 22. Further, as another method, the gas generating agent may be disposed in the igniter 22 as disclosed in WO 01-031282, JP 2003-25950 A, and the like. As one example of the gas generating agent, there may be exemplified a single base smokeless explosive formed of 98 mass% of nitrocellulose, 0.8 mass% of diphenylamine, and 1.2 mass% of potassium sulfate. Further, various types of gas generating agents used in a gas generator for an air bag and a gas generator for a seat belt pretensioner may be used. A combustion completion time period of the gas generating agent can be changed by adjusting a dimension, a size, a shape, and particularly, a surface shape of the gas generating agent at the time of being disposed in the combustion chamber 20a or the like. With this, the pressure applied to the piston 40 can be adjusted to a desired pressure.

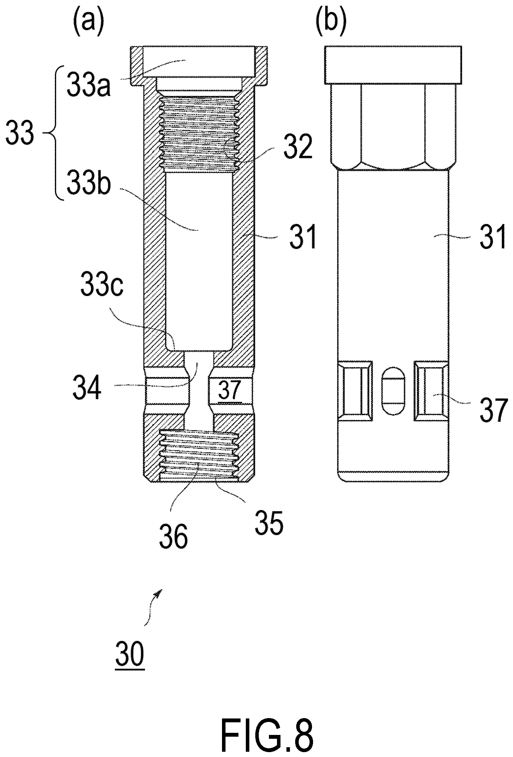

Next, the attachment 30 will be described based on FIG. 8. Note that FIG. 8 includes the diagram (a) on the left side that is a cross-sectional view of the attachment 30, and the diagram (b) on the right side that is an external view of the attachment 30. The attachment 30 is a member for attaching the actuator 20, the plunger 80, and the container 70 as illustrated in FIG. 5.

The internal space of the body 31 includes a first region 33, extending from the base end side to the center, where the actuator 20 is disposed as illustrated in FIG. 5. The first region 33 includes a region 33a on the base end side where the base end portion 21c of the actuator 20 is generally positioned, and a region 33b on the distal end side of the first region 33 where the center portion 21a and the distal end portion 21b of the actuator 20 are generally positioned. The region 33b has a smaller diameter than the region 33a. The female thread portion 32 is disposed on the inner wall surface at a portion of the region 33b close to the region 33a. The female thread portion 32 is formed so as to engage with the male thread portion 26 provided on the center portion 21a of the actuator 20.

The internal space of the body 31 further includes a second region 34 in communication with the first region 33. The second region 34 is a region in which the plunger 80 is generally disposed as illustrated in FIG. 5, and is a hollow region formed in a cylindrical shape extending along the axial direction of the body 31. The second region 34 has one end in communication with the region 33b of the first region 33. The second region 34 has a diameter smaller that is smaller than the diameter of the region 33b, and enables a sliding movement of the plunger 80. A through hole 37 extends from a side outer surface of the attachment 30 to the second region 34, to be formed through the body 31. Through the through hole 37, the user can check the status (such as whether the injector assembly 10 is before or after being actuated, for example) of the plunger 80 in the injector assembly 10 from the outside (see FIG. 1).

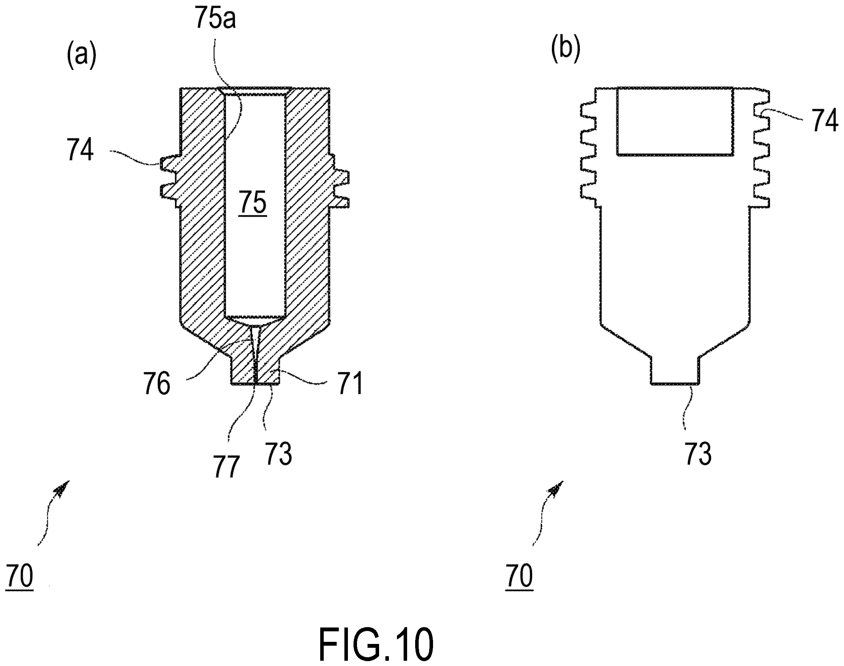

The internal space of the body 31 further includes a third region 35 in communication with the second region 34. The third region 35 is a region in which a part of the container 70 is generally disposed as illustrated in FIG. 5, and has one end in communication with the second region 34, and has the other end open to the distal end surface of the attachment 30. A female thread portion 36 for attachment to the container 70 is formed in the third region 35. The female thread portion 36 is screwed with a male thread portion 74 of the container 70 illustrated in FIG. 10 described below, whereby the attachment 30 and the container 70 are coupled to each other.

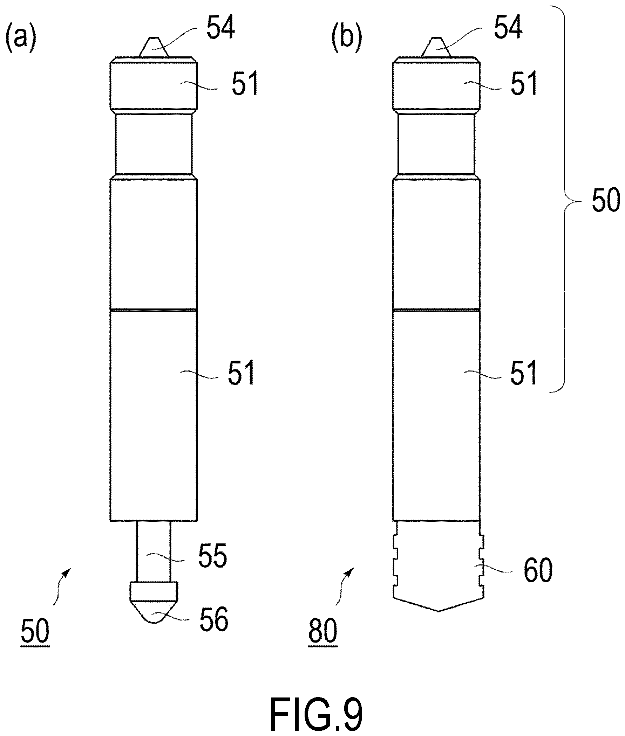

Next, the plunger 80 will be described based on FIG. 9. FIG. 9 includes the diagram (a) on the left side that is an external view of a plunger rod 50, which is one of the components of the plunger 80, and a diagram (b) on the right side that is an external view of the plunger 80. The plunger 80 is a member that pressurizes the ejection solution by energy received from the piston 40, and for the plunger rod 50, it is possible to use a resin material suitable for pressurization, for example, a resin material similar to that used for the attachment 30, known nylon 6-12, polyarylate, polybutylene terephthalate, polyphenylene sulfide, liquid crystal polymer, or the like. Further, a filler such as glass fibers and glass filler may be contained in those resins. From 20 to 80 mass% of glass fibers may be contained in polybutylene terephthalate, from 20 to 80 mass% of glass fibers may be contained in polyphenylene sulphide, or from 20 to 80 mass% of minerals may be contained in a liquid crystal polymer. The plunger rod 50 includes a shaft member 51, and has a base end side end surface provided with a protrusion 54. The protrusion 54 is shaped and sized to be capable of fitting in the recess portion 44 of the shaft member of the piston 40 of the actuator 20, when the plunger 80 is incorporated in the injector assembly 10.

Further, in the plunger rod 50, a protrusion 56 is provided to a distal end side of the shaft member 51 with a neck portion 55 with a smaller diameter than the shaft member 51 provided in between. The protrusion 56 is shaped like a weight to have a diameter being greater than the diameter of the neck portion 55 near a portion to be connected with the neck portion 55 and reducing toward the distal end side. The maximum diameter of the protrusion 56 is smaller than the diameter of the shaft member 51. A stopper portion 60 formed of an elastic member such as rubber is attached to the neck portion 55 and the protrusion 56, whereby the plunger 80 is formed (see FIG. 9(b)). An attachment hole (not illustrated) is formed in the stopper portion 60, and engages with the neck portion 55 and the protrusion 56, so that the stopper portion 60 is less likely to be detached from the plunger rod 50.

Specific examples of materials of the stopper portion 60 include butyl rubber and silicon rubber. Further, there may be exemplified a styrene-based elastomer or a hydrogenated styrene-based elastomer, or a substance obtained by mixing a styrene-based elastomer or a hydrogenated styrene-based elastomer with polyolefin such as polyethylene, polypropylene, polybutene, and an α-olefin copolymer, oil such as liquid paraffin and process oil, or a powder inorganic substance such as talc, cast, and mica. Further, as the material of the stopper portion 60, a fluorine-based elastomer such as vinylidene fluoride-hexafluoropropylene copolymer, a polyvinyl chloride-based elastomer, an olefin-based elastomer, a polyester-based elastomer, a polyamide-based elastomer, a polyurethane-based elastomer, various rubber materials (particularly, a vulcanized material) such as natural rubber, isoprene rubber, chloroprene rubber, nitrile butadiene rubber, and styrene-butadiene rubber, or a mixture thereof may be employed. Furthermore, the stopper portion 60 pressurizes the ejection solution by sliding within the container 70 described below. Thus, a surface of the stopper portion 60 and an inner wall surface 75a of the accommodating space 75 of the container 70 may be coated or processed using various matters, to guarantee/adjust slidability between the stopper portion 60 and the inner wall surface 75a of the accommodating space 75 of the container 70. Examples of the coating agent may include polytetrafluoroethylene (PTFE), silicon oil, diamond-like carbon, nano diamond, and the like.

Next, the container 70 will be described based on FIG. 10. Note that FIG. 10 includes the diagram (a) on the left side that is a cross-sectional view of the container 70, and the diagram (b) on the right side that is an external view of the container 70. The container 70 is a member containing an ejection solution to be pressurized by the plunger 80, and is a member that defines a flow path for injecting the pressurized ejection solution to the target region. In consideration of this point, a resin material forming the container 70 can be adopted, and, for example, in addition to a resin material similar to that used for the attachment 30, nylon 6-12, polyarylate, polybutylene terephthalate, polyphenylene sulphide, a liquid crystal polymer, or the like can be used. Further, a filler such as glass fibers and glass filler may be contained in those resins. From 20 to 80 mass% of glass fibers may be contained in polybutylene terephthalate, from 20 to 80 mass% of glass fibers may be contained in polyphenylene sulphide, or from 20 to 80 mass% of minerals may be contained in a liquid crystal polymer.

The container 70 includes an accommodating space 75, in which the stopper portion 60 of the plunger 80 are movable, accommodating the ejection solution, and a nozzle portion 71 including a flow path 76 connecting the accommodating space 75 to the outside of the container 70. The nozzle portion 71 has a columnar outer circumference on the distal end side. Note that in the injector assembly 10, as illustrated in FIG. 5, a positional relationship between the plunger 80 and the container 70 is determined so that the stopper portion 60 of the plunger 80 can slide within the accommodating space 75 in a direction toward the nozzle portion 71 (direction toward the distal end side). The ejection solution is sealed in a space defined by stopper portion 60 of the plunger 80 and the container 70. The flow path of the container 70 opens in a distal end surface 73 of the nozzle portion 71, so that the ejection port 77 is formed. Thus, when the plunger 80 slides within the accommodating space 75, the ejection solution accommodated in the accommodating space 75 is pressurized to be ejected from the ejection port 77 through the flow path 76.

The flow path 76 provided in the container 70 has a diameter smaller than the inner diameter of the accommodating space 75. With this configuration, the ejection solution that has been applied with a high pressure is ejected to the outside through the ejection port 77. The male thread portion 74 for attaching the container 70 to the attachment 30 is formed on the base end side of the container 70. The male thread portion 74 is screwed with the female thread portion 36 of the attachment 30.

Note that the profile on the distal end side of the stopper portion 60 of the plunger 80 is shaped to substantially match the profile of the inner wall surface 75a near a portion where the accommodating space 75 and the flow path 76 are connected to each other (the deepest part of the accommodating space 75). With this configuration, a smallest possible gap can be formed between the stopper portion 60 and the inner wall surface 75a of the container 70 when the plunger 80 slides for ejecting the ejection solution and reaches the deepest part of the accommodating space 75, whereby the ejection solution can be prevented from wastefully remaining in the accommodating space 75. However, the shape of the stopper portion 60 is not limited to a particular shape as long as desired effects can be obtained with the injector 1 according to the present embodiment.

Now, how the injector assembly 10 is assembled will be described. In a state where the stopper portion 60 of the plunger 80 is inserted to the deepest part of the accommodating space 75 of the container 70, the plunger 80 is retracted with the ejection port 77 of the container 70 in communication with the ejection solution. The stopper portion 60 and the inner wall surface 75a of the accommodating space 75 are suitably in close contact with each other, the retraction action will produce negative pressure in the accommodating space. Thus, the accommodating space 75 can be filled with the ejection solution through the ejection port 77. In this process, the plunger 80 is retracted to an extent enough for making the part of the plunger 80 (plunger rod 50) protruding from the container 70 pass through the second region 34 to reach the first region 33 (the region 33b illustrated in FIG. 8), when the container 70 is attached to the attachment 30 in this state.

After the container 70 filled with ejection solution in the accommodating space 75 is attached to the attachment 30, the actuator 20 is inserted to the attachment 30 from the side of the first region 33. The actuator 20 is inserted until the distal end surface of its distal end portion 21b comes into contact with a distal end surface 33c of the region 33b of the attachment 30 (see FIG. 8). Then, in this process, the male thread portion 26 provided to the center portion 21a of the actuator 20 is screwed with the female thread portion 32 of the attachment 30, whereby the actuator 20 and the attachment 30 are suitably coupled to each other. Furthermore, in this process, the recess portion 44 of the shaft member 41 of the piston 40, which is incorporated in the actuator 20, engages with the protrusion 54 of the shaft member 51 of the plunger 80, and the plunger 80 is pushed by the piston 40 toward the distal end side. Note that, a fixing force of the piston 40 in the distal end portion 21b of the actuator 20 is set to an extent that the piston 40 can slide in the distal end portion 21b in a sufficiently smooth manner by a pressure received from the combustion product produced by the igniter 22, and to an extent that the piston 40 can suitably resist force received from the plunger 80 so that the position of the piston 40 is not displaced when the injector assembly 10 is assembled. Alternatively, a stopper may be formed at an intended position of the piston 40, so that the top surface of the first flange 42 of the piston 40 faces the combustion chamber 20a of the actuator 20 and is not displaced toward the combustion chamber 20a as illustrated in FIG. 6.

Thus, when the actuator 20 is attached to the attachment 30 to which the container 70 and plunger 80 are attached as described above, the plunger 80 is pushed to move from the piston 40 toward the distal end side, whereby the plunger 80 is positioned at a predetermined position within the container 70. Note that, in response to pressing of the plunger 80, a part of the ejection solution is discharged from the ejection port 77.

When the plunger 80 is thus positioned at the final position as described above, formation of the injector assembly 10 is completed. In this injector assembly 10, the position of the stopper portion 60 of the plunger 80 in the accommodating space 75 of the container 70 is mechanically determined. The final position of the stopper portion 60 is a position uniquely determined in the injector assembly 10, and hence an amount of the ejection solution that is finally stored in the accommodating space 75 in the injector assembly 10 can be a predetermined amount determined in advance.

The injector assembly 10 thus configured can be loaded into the housing 2 with the ignition pin 22b of the igniter 22 fitted into the socket 7 on the housing 2, whereby the injector 1 is prepared to be usable (see FIGS. 1 to 3). The user holds the housing 2 of such injector 1 with one hand and slides the first switch 5 located on the back side of the housing 2 for a predetermined period of time, putting the injector 1 in the standby state. In this state, when the user presses the second switch 6 with the ejection port 77 being in contact with the target region, the igniter 22 is actuated, and the ejection solution is pressurized via the piston 40 and the plunger 80. Thus, the ejection is implemented, and the ejection solution is injected into the target region.

### Examples

Each of the configurations, combinations thereof, and the like in each embodiment are an example, and various additions, omissions, substitutions, and other changes may be made as appropriate without departing from the spirit of the present invention. The present disclosure is not limited by the embodiments and is limited only by the claims.

### <Example 1>

### (Manufacturing of attachment)

A pellet of a mixture of a polycarbonate resin and ABS resin which has an ABS resin content of 10 mass% (trade name: NOVALLOY S69P1, available from Daicel Polymer Ltd.) was provided as the resin material and dried for three hours or longer at 80°C. In accordance with ISO178, the flexural modulus of NOVALLOY S69P1 measured at 23°C and 50% RH is 2640 MPa (catalogue value).

Next, using an injection molding machine (trade name: SE130EV, available from Sumitomo Heavy Industries, Ltd.) and a mold for the attachment of the injector illustrated in FIG. 3, molding was performed at a cylinder temperature of 250°C and a mold temperature of 60°C to obtain an attachment.

### (Evaluation of crack resistance)

The injector of FIG. 3 (nozzle diameter: diameter 0.1 mm) equipped with the obtained attachment was filled with 100 µL water, and the water was ejected from the ejection port by combustion of an ignition charge.

190 mg of an explosive (ZPP) containing zirconium and potassium perchlorate was used as the ignition charge, and 40 mg of a single base smokeless explosive (hereinafter, also referred to as "GG") was used as a gas generating agent.

The attachment after ejection was visually evaluated based on the following evaluation criteria: The evaluation results were indicated in Table 1.
o: No crack was observed, and there was no change as compared with before ejection.
×: Crack was generated and the attachment was broken.

### Example 2

An attachment was manufactured in the same manner as in Example 1 except that a mixture of a polycarbonate resin and ABS resin which has an ABS resin content of 20 mass% (trade name: NOVALLOY S6700, available from Daicel Polymer Ltd.) was used as the resin material. In accordance with ISO178, the flexural modulus of NOVALLOY S6700 measured at 23°C and 50% RH is 2800 MPa (catalogue value).

The crack resistance was evaluated for the obtained attachment, and the results were shown in Table 1.

### Example 3

An attachment was manufactured in the same manner as in Example 1 except that a mixture of a polycarbonate resin and ABS resin which has an ABS resin content of 30 mass% (trade name: NOVALLOY S6500L, available from Daicel Polymer Ltd.) was used as the resin material. In accordance with ISO178, the flexural modulus of NOVALLOY S6500L measured at 23°C and 50% RH is 2600 MPa (catalogue value).

The crack resistance was evaluated for the obtained attachment, and the results were shown in Table 1.

### <Comparative Example 1>

An attachment was manufactured in the same manner as in Example 1 except that a polycarbonate resin (trade name: Iupilon EB-3001R, available from Mitsubishi Engineering-Plastics Corporation) was used as the resin material. In accordance with ISO178, the flexural modulus of Iupilon EB-3001R measured at 23°C and 50% RH is 2300 MPa (catalogue value).

The crack resistance was evaluated for the obtained attachment, and the results were shown in Table 1.

### [Table 1]

**Table 1**

| | Resin material | | Crack resistance |
|---|---|---|---|
| | Resin component | Flexural modulus (MPa) | |
| Comparative Example 1 | Iupilon EB-3001R | 2300 | × |
| | (Polycarbonate resin 100 mass%) | | |
| Example 1 | NOVALLOY S69P1 | 2640 | o |
| | (Polycarbonate resin-ABS resin mixture) | | |
| | (ABS resin content: 10 mass%) | | |
| Example 2 | NOVALLOY S6700 | 2800 | ο |
| | (Polycarbonate resin-ABS resin mixture) | | |
| | (ABS resin content: 20 mass%) | | |
| Example 3 | NOVALLOY S6500L | 2600 | ο |
| | (Polycarbonate resin-ABS resin mixture) | | |
| | (ABS resin content: 30 mass%) | | |

From the evaluation results shown in Table 1, it was found that even when the attachment formed from a resin material having a flexural modulus of 2600 MPa or greater was imparted with an instantaneous impact load based on the occurrence of the combustion energy of the ignition charge in the actuator, crack generation was not observed, and the attachment had sufficient crack resistance.

On the other hand, it was found that in the attachment of Comparative Example 1 formed from a resin material having a flexural modulus of less than 2600 MPa, as a result of crack generation, the attachment was broken, and the crack resistance was not sufficient.

### Reference Signs List

- 1: Injector
- 2: Housing
- 2a: Grip portion
- 3: Power cable
- 4: Connector
- 5: First switch
- 6: Second switch
- 10: Injector assembly
- 20: Actuator
- 21: Body
- 22: Igniter
- 30: Attachment
- 31: Body
- 32: Female thread portion
- 33: First region
- 34: Second region
- 35: Third region
- 36: Female thread portion
- 37: Through hole
- 40: Piston
- 50: Plunger rod
- 51: Shaft member
- 60: Stopper portion
- 70: Container
- 71: Nozzle portion
- 75: Accommodating space
- 76: Flow path
- 77: Ejection port
- 80: Plunger

## Claims

1. A crack resistant member comprising:
a member body;
a first region that is a hollow space provided in the member body to open to a surface of the member body and is configured for disposing a driving part that outputs a pressing force in an extension direction of the space; and
a coupling portion that is configured for mechanically coupling to the driving part and is formed on an inner wall surface of the first region,
wherein at least the coupling portion is formed from a resin material having a flexural modulus of 2600 MPa or greater as measured in accordance with ISO 178.

2. The crack resistant member according to claim 1, wherein the driving part includes a drive source that contains predetermined energy for generating the pressing force, and is configured to generate the pressing force by releasing the predetermined energy to outside.

3. The crack resistant member according to claim 2, wherein the drive source is an ignition charge, and
the driving part is configured to generate the pressing force by releasing combustion energy which is caused by combustion of the ignition charge and is the predetermined energy.

4. The crack resistant member according to claim 2 or 3, wherein the driving part is configured to complete the release of the predetermined energy within 10 msec from start of actuation of the driving part.

5. The crack resistant member according to any one of claims 1 to 4, wherein the coupling portion is a screw portion formed on the inner wall surface of the first region along an extension direction of the first region.

6. The crack resistant member according to any one of claims 1 to 5, wherein the resin material contains one or more selected from the group consisting of a mixture of a polycarbonate resin and an acrylonitrile-butadiene-styrene copolymer (ABS resin), a polyolefin-based resin, a crystalline polyamide resin, and a crystalline polyester resin.

7. The crack resistant member according to claim 6, wherein in the mixture of the polycarbonate resin and the ABS resin, a proportion of the ABS resin relative to a total amount of the polycarbonate resin and the ABS resin is 10 mass% or greater and 30 mass% or less.

8. A needleless injector configured to eject an intended injection substance to a target region without using an injection needle, the needleless injector comprising:
an attachment that is the crack resistant member described in any one of claims 1 to 7 and couples an actuator, which is the driving part, into the attachment with the coupling portion;
the actuator;
a housing part that has an accommodating space that accommodates the intended injection substance and defines a flow path from the accommodating space in such a manner that the intended injection substance is ejected to the target region;
a metal piston which is arranged to propel inside the needleless injector in a predetermined direction when the pressing force is applied by actuation of the driving part; and
a plunger that defines the accommodating space and propels inside the housing part by the pressing force to pressurize the intended injection substance.
